(19) Europäisches Patentamt
European Patent Office
Office européen des brevets

(11) Numéro de publication: **0 678 579 A1**

(12) **DEMANDE DE BREVET EUROPEEN**

(21) Numéro de dépôt: **94400863.0**

(22) Date de dépôt: **21.04.94**

(51) Int. Cl.6: **C12P 41/00**, C07C 62/14, C12P 7/40

(43) Date de publication de la demande:
**25.10.95 Bulletin 95/43**

(84) Etats contractants désignés:
**IT**

(71) Demandeur: **MIDY S.p.A.**
**Via Piranesi 38**
**I-20137 Milano (IT)**

(72) Inventeur: **Cecchi, Robert**
**Viale dei Tigli, 1**
**I-20075 Lodi (Milano) (IT)**
Inventeur: **Barzaghi, Laura**
**Via E. Borsa, 51**
**I-20052 Monza (Milano) (IT)**
Inventeur: **Guzzi, Umberto**
**c/o Midy S.p.A.**
**38 Via Piranesi**
**20137 Milano (IT)**

(74) Mandataire: **Gillard, Marie-Louise et al**
**Cabinet Beau de Loménie**
**158, rue de l'Université**
**F-75340 Paris Cédex 07 (FR)**

(54) **Procédé enzymatique pour la préparation de dérivés tétraliniques optiquement actifs.**

(57) La présente invention concerne un procédé enzymatique pour la préparation d'acides 2-tétrahydronaphtoiques optiquement actifs à partir des esters racémates correspondants par réaction avec une lipase.

EP 0 678 579 A1

La présente invention concerne un procédé pour la préparation de dérivés optiquement actifs de la tétraline.

Dans les dix dernières années, l'application des enzymes dans la synthèse enantiosélective a été amplement étudiée (Klibanov, Acc. Chem. Res., 23 : 114,1990). Plus particulièrement, l'enzyme lipase a été utilisée dans des estérifications et transestérifications asymétriques, notamment dans la préparation d'alcools, esters et acides.

De nombreux dérivés à structure tétralinique, sous forme optiquement active, sont décrits en littérature, notamment en tant qu'intermédiaires de synthèse (EP-A-436435, EP-B-347313, EP-A-211721, DE 2803582, EP-A-334538) ou comme outils de laboratoire dans les essais pharmacologiques et biochimiques. Par exemple, la 8-hydroxy-2-diisopropylaminotétraline (8-OH-DPAT) est utilisée comme produit de référence dans les tests sur la sérotonine, notamment en tant que $5HT_{1A}$ agoniste (J. Med. Chem., 1989, 32 : 779-783 ; Eur. J. Med. Chem., 1991, 26 : 215-220).

Plus particulièrement EP-A-436435 décrit des acides 1,2,3,4-tétrahydro-2-naphtoïques méthoxysubstitués, sous forme optiquement active. La séparation des énantiomères de ces acides à partir du racémate, lorsqu'elle est effectuée par les méthodes chimiques habituelles, est non seulement laborieuse mais aussi peu performante et les résultats, en termes de pureté énantiomérique des produits séparés, sont parfois peu satisfaisants.

On a maintenant trouvé qu'en se basant sur la cinétique d'une hydrolyse enzymatique d'esters alkyliques d'acides tétralinecarboxyliques, on peut effectuer la résolution des énantiomères par une réaction très simple et performante.

Notamment, il a été trouvé qu'en soumettant un ester 1,2,3,4-tétrahydro-2-naphtoïque méthoxy-substitué à l'action de la lipase, l'enzyme hydrolyse préférablement la forme *(R)* de l'ester, en laissant la forme *(S)* presque inaltérée, en permettant ainsi de séparer les deux formes.

La présente invention a donc pour objet un procédé de préparation des composés de formule (I) sous forme optiquement active

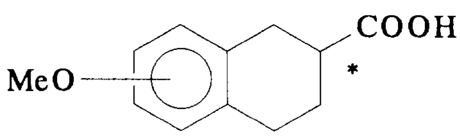

(I)

caractérisé en ce que :

(a) on soumet un ester racémique de formule

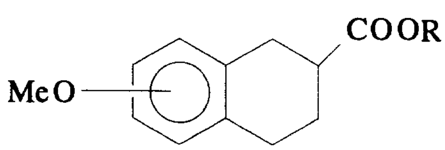

(II)

dans laquelle R est un alkyle en $C_1$-$C_3$, à une hydrolyse par une lipase ; puis

(b) lorsque environ 50 % de l'ester est hydrolysé en acide, on interrompt l'hydrolyse par inactivation de l'enzyme et on récupère l'ester de configuration *(S)* de formule

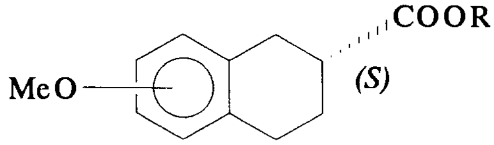

(II')

qui n'a pas réagi, enfin

(c) soit on hydrolyse ledit ester de formule (II') et on isole l'acide de configuration *(S)* de formule

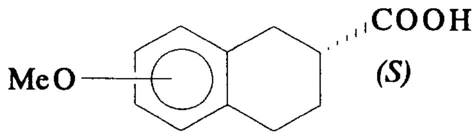

(S) (I')

soit on isole l'acide de configuration *(R)* de formule

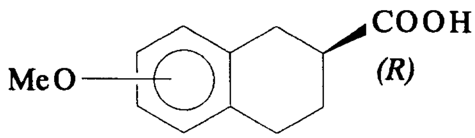

(R) (I'')

tel qu'obtenu de la réaction d'hydrolyse lipasique, après interruption de l'hydrolyse à l'étape (b).

Dans les formules (I), (II), (I'), (I'') et (II') ci-dessus et dans la description ci-après, Me désigne le groupe méthyle.

Les esters de formule (II) sont décrits par exemple dans EP-A-300404, EP-A-436435, Synthesis 9:727-9, (1983), J. Am. Chem. Soc., 104:7609-22, (1982), ou bien ils peuvent être aisément préparés par estérification des acides racémiques correspondants. Parmi ces produits de départ ceux de formule (II) où R est méthyle ou éthyle sont particulièrement préférés.

La lipase utilisée dans la réaction de l'étape (a) est la lipase de pancréas de porc (PPL de l'anglais "porcine pancreatic lipase"), qui est une enzyme vendue dans le commerce sous forme fraîche ou lyophilisée (de préférence la lipase utilisée est la lipase de pancréas de porc - Type II, Sigma L-3126). L'enzyme lipase peut éventuellement être immobilisée, par des liaisons covalentes, sur une matrice polymérique, par exemple une résine selon les techniques décrites par exemple par S. Fukuy, ENZYMES ENG., 6:191-200 (1982).

La réaction d'hydrolyse énantiosélective de l'étape (a) est effectuée de préférence en milieu aqueux, en mélange avec un solvant organique miscible dans l'eau et en présence d'un système tampon. Plus particulièrement, le mélange réactionnel est tamponné par un tampon à un pH d'environ 7, préparé selon des méthodes connues tel que par exemple le tampon phosphate. Ce pH correspond au pH des performances optimales de l'enzyme.

La température de réaction de l'étape (a) peut être librement choisie dans un intervalle de température dans lequel l'enzyme n'est pas desactivée, généralement allant de 0° C à 60° C, avantageusement de 5° C à 40° C, de préférence de 10° à 30° C, normalement à la température ambiante.

Le solvant organique utilisé dans l'étape (a) du procédé de l'invention est un solvant miscible dans l'eau, tel que par exemple un alcool, comme le méthanol, l'éthanol, le n-propanol, l'*iso*-propanol, le n-butanol, le *sec*-butanol, l'*iso*-butanol, le *tert*-butanol ; une cétone, comme l'acétone ; un éther cyclique, comme le tétrahydrofuranne ou le dioxanne; le *tert*-butanol étant un solvant particulièrement avantageux. La quantité dudit solvant n'est pas un paramètre critique ; généralement, on emploie une quantité de solvant suffisante à dissoudre l'ester de départ.

Normalement, l'ester racémique de formule (II) est dissous dans un solvant organique miscible dans l'eau et la solution obtenue est ajoutée à un tampon à pH7. A ce mélange est ensuite ajoutée l'enzyme PPL qui catalyse l'hydrolyse sélective de l'énantiomère *(R)* de l'ester. La réaction est contrôlée par addition d'une base, notamment NaOH, pour maintenir la valeur du pH presque constante à un pH d'environ 7. La réaction est terminée lorsque l'enzyme a hydrolysé la moitié de l'ester, notamment l'isomère *(R)* de l'ester, à savoir quand on a ajouté une quantité de base égale au maximum à la moitié d'équivalents-mole de l'ester de départ.

Dans l'étape (b), pour arrêter la réaction, on inactive l'enzyme, par exemple par addition d'une base, de façon à augmenter le pH de la solution jusqu'à une valeur basique, de préférence d'au moins environ 8. La base utilisée peut être inorganique, comme un hydroxyde alcalin, notamment de sodium ou de potassium, un carbonate alcalin, notamment de sodium ou de potassium ou une amine, de préférence tertiaire, telle que la triéthylamine.

L'isomère ayant la configuration *(S)* de l'ester, de formule (II'), qui n'a pas été hydrolysé par la lipase, est extrait dans un solvant organique immiscible à l'eau et isolé selon les techniques habituelles, par exemple par évaporation du solvant sous pression réduite.

La phase aqueuse résiduelle renferme l'acide libre correspondant à l'ester de départ de formule (II) de configuration absolue *(R)*.

Dans l'étape (c), on procède à la séparation des deux énantiomères *(S)* et *(R)*.

Pour isoler l'isomère *(S)* de formule (I'), on soumet l'ester méthylique (II') à une hydrolyse en conditions acides ou basiques. L'hydrolyse en conditions basiques correspond à une saponification effectuée selon les techniques habituelles, par exemple avec un hydroxyde alcalin tel que l'hydroxyde de sodium ou de potassium. L'acide (I') est isolé selon les techniques conventionnelles, par acidification avec un acide minéral ou organique, par exemple à l'aide de l'acide chlorhydrique ou sulfurique. L'hydrolyse en conditions acides est conduite avec un acide tel que l'acide chlorhydrique ou sulfurique et l'énantiomère *(S)* de formule (I') est isolé par neutralisation avec une base telle que l'hydroxyde de sodium.

Pour l'obtention de l'énantiomère *(R)* de formule (I''), ledit acide, qui constitue le produit de l'hydrolyse par la lipase de l'étape (a) et qui reste dans la phase aqueuse après la séparation de l'ester (II'), est récupéré et isolé selon les méthodes conventionnelles, notamment par acidification de la solution aqueuse et filtration du précipité formé.

Lorsque l'on désire obtenir les dérivés (I'') de configuration absolue *(R)*, il est avantageux d'arrêter la réaction de l'étape (a) juste avant que 50 % de l'ester soit hydrolysé, à savoir avant que tout l'isomère *(R)* de l'ester réagisse. Cela permet d'obtenir l'acide *(R)* de formule (I'') pur, avant que la réaction parasite d'hydrolyse de l'isomère *(S)* de l'ester de formule (II) intervienne.

Si, au contraire, on souhaite obtenir les dérivés de configuration absolue *(S)*, il est convenable de poursuivre l'hydrolyse un peu au-delà de la consommation de 50 % de l'ester (II), de façon à être certain que tout l'isomère *(R)* est hydrolysé par l'enzyme et que tout l'ester résiduel a donc la configuration *(S)*.

Ainsi, selon un aspect préféré de la présente invention, dans l'étape (b) on interrompt l'hydrolyse enzymatique juste avant que 50% du composé (II) soit hydrolysé, par addition d'hydroxyde de sodium jusqu'à pH basique, on extrait le composé (II') qui n'a pas réagi dans un solvant organique immiscible à l'eau puis on élimine la phase organique et, dans l'étape (c) on traite la phase aqueuse avec un acide minéral ou organique et on sépare l'acide de configuration *(R)* de formule (I'') ainsi précipité.

Selon un autre aspect préféré de la présente invention, dans l'étape (b) on interrompt l'hydrolyse enzymatique juste après que 50% du composé (II) soit hydrolysé, par addition d'hydroxyde de sodium jusqu'à pH basique, on extrait le composé (II') qui n'a pas réagi dans un solvant organique immiscible à l'eau puis on élimine la phase aqueuse, on isole l'ester (II') et, dans l'étape (c) on hydrolyse ledit ester de formule (II') et on isole l'acide de configuration *(S)* de formule (I').

Les acides isomères *(R)* et *(S)* sont obtenus par le procédé de la présente invention sous forme optiquement pure, les excès énantiomériques étant de loin supérieurs à 90 %.

Les esters (II') qui peuvent être isolés sous forme extrêmement pure sont des produits nouveaux et constituent un objet ultérieur de la présente invention. Parmi ces composés, ceux de formule (II') où MeO est en position 6 ou 7 et R représente méthyle sont particulièrement préférés.

Les composés de formule (I') et (I'') sont des intermédiaires de réaction très versatiles, utiles dans la synthèse de nombreux dérivés à structure tétralinique.

Plus particulièrement, les composés de formule (I') sont décrits en tant qu'intermédiaires dans les synthèses des amines optiquement actives de formule (III)

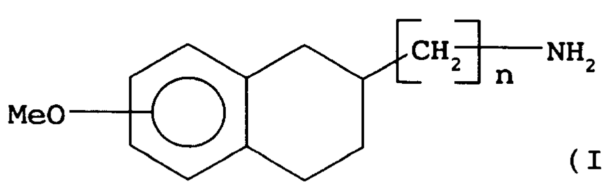

(III)

dans laquelle n est 0 ou 1.

Le procédé pour la préparation des amines de formule (III) ci-dessus est caractérisé en ce que l'on conduit une cinétique d'hydrolyse enzymatique sur l'ester racémate de formule (II)

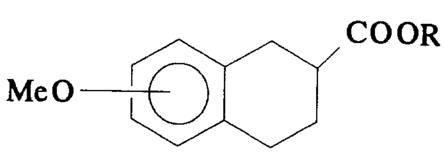

(II)

dans laquelle R est défini comme ci-dessus, à l'aide de la lipase de pancréas de porc, selon la méthode décrite dans les étapes (a) (b) et (c) ci-dessus et on transforme ensuite les acides isomères de formule (I')

et (I'') ainsi obtenus en amines de formule (III) selon les méthodes décrites dans EP-A-436435, notamment par la réaction de Curtius directement sur l'acide optiquement actif peur les composés de formule (III) où n = 0 et par réduction de l'amide obtenue à partir de l'acide optiquement actif pour les composés où n = 1.

Evidemment, lorsque l'on veut obtenir les amines de formule (III) à configuration absolue *(S)*, on utilisera comme acide de départ celui de formule (I') ; si l'on veut obtenir les amines de formule (III) à configuration absolue *(R)*, on utilisera l'acide de formule (I'') en tant que produit de départ.

Les exemples qui suivent illustrent mieux l'invention.

Les excès énantiomériques (e.e) ont été calculés sur la base des données de HPLC, dont l'analyse est conduite dans les conditions suivantes:

(a) - Colonne : CHIRALCEL OD,
- phase mobile : mélange hexane/acide trifluroacétique = 100/1.
- flux : 1,5 ml/min.

Temps de rétention (min.):
- Acide 6-méthoxy-1,2,3,4-tétrahydro-(2R)2-naphtoïque = 20,3
- Acide 6-méthoxy-1,2,3,4-tétrahydro-(2S)2-naphtoïque = 23,5
- Méthyl 6-méthoxy - 1,2,3,4-tétrahydro-(2R)2-naphtoate = 12,95
- Méthyl 6-méthoxy - 1,2,3,4-tétrahydro-(2S)2-naphtoate = 14,3
- Acide 7-méthoxy - 1,2,3,4-tétrahydro-(2R)2-naphtoïque = 19,2
- Acide 7-méthoxy - 1,2,3,4-tétrahydro-(2S)2-naphtoïque = 16,8

(b) - Colonne : CHIRALCEL OD,
- phase mobile : mélange hexane/isopropanol = 100/2.
- flux : 0,5 ml/min.

Temps de rétention (min.):
- Méthyl 7-méthoxy- 1,2,3,4-tétrahydro-(2R)2-naphtoate = 19,5
- Méthyl 7-méthoxy- 1,2,3,4-tétrahydro-(2S)2-naphtoate = 22,6

(c) - Colonne : CHIRALCEL OD,
- phase mobile : mélange hexane/isopropanol = 85/5
- flux : 1 ml/min.

Temps de rétention (min.):
- 6-méthoxy-1,2,3,4-tétrahydronaphtalen-(2R)2-carboxamide = 8,96

La formule pour calculer les excès énantiomériques est la suivante:

$$\text{e.e.} = \frac{A1 - A2}{A1 + A2}$$

dans laquelle A1 et A2 représentent les aires correspondantes aux deux isomères *(R)* et *(S)* obtenues par l'analyse HPLC.

La solution tampon utilisée dans les exemples qui suivent est un tampon phosphate commercialisé par la société Merck sous le code *Merck 9439*.

## Exemple 1

### Acide 6-méthoxy-1,2,3,4-tétrahydro-(2R)2-naphtoïque.

On dissout 3 g (0,0136 mole) de méthyl 6-méthoxy-1,2,3,4-tétrahydro-2-naphtoate dans 100 ml de *tert*-butanol et on ajoute à la solution ainsi préparée 300 ml de tampon phosphate à pH 7. La valeur du pH de la solution, qui augmente jusqu'à 7,3 - 7,5, est ramenée à 7,1 par addition de HCl 1N. On ajoute au mélange 1,5 g d'enzyme PPL Sigma (indiquée comme "porcine pancreatic lipase" type II, crude, Sigma L-3126 - 50 U/mg using triacetine). Au fur et à mesure que la réaction s'effectue, le pH tend à baisser mais on le maintient constant par addition d'une solution 0,25 N de NaOH en controlant le pH par un appareil automatique de titration. Après 4-5 heures, lorsque environ 22 ml de NaOH 0,25 N ont été consommés, on ajoute à la solution du bicarbonate de sodium jusqu'à obtenir pH 8, pour arrêter la réaction par inactivation de l'enzyme. On extrait dans de l'éther éthylique et on sépare les deux phases. La phase organique contient l'ester, principalement de configuration *(S)* qui n'a pas réagi et qui peut être récupéré comme indiqué dans l'Exemple 2 ci-dessous. On acidifie la phase aqueuse à l'aide d'acide sulfurique concentré et on filtre le précipité formé. On obtient ainsi 1,16 g de l'acide indiqué en titre qu'on cristallise dans l'acétate d'éthyle. P.f. 130-131 °C ; $[\alpha]_{20}^{D}$ = +57,8° (c. 1,4%, $CHCl_3$); e.e. 93,7 % (HPLC effectuée dans les

conditions (a)). Ce produit est plus pur que celui décrit dans EP-A-436 435, préparation (O)(i). Deux autres préparations effectuées dans les mêmes conditions ont donné le même produit ayant un e.e. de 92% et 93,1 % (HPLC effectuée dans les conditions (a)).

## Exemple 2

### Acide 6-méthoxy-1,2,3,4-tétrahydro-(2S) 2-naphtoïque

La phase organique résiduelle de l'Exemple 1 est séchée sur du sulfate de sodium et évaporée sous pression réduite et on distille ensuite l'ester (Eb = 128-132 °C à 0,1 mbar) contenant principalement l'isomère *(S)* (e.e. 68,9 % HPLC effectuée dans les conditions (a)). On dissout 5 g (0,022 mole) de l'ester ainsi recupéré dans 175 ml de *tert*-butanol; on ajoute à la solution ainsi obtenue 500 ml de tampon phosphate à pH7. On porte à pH 7,04 par addition d'acide sulfurique à 5% et on ajoute 2,5 g de PPL Sigma (indiquée comme "porcine pancreatic lipase" type II, crude, Sigma L-3126 - 39 U/mg using triacetine). Au fur et à mesure que la réaction s'effectue, le pH baisse; on le maintient constant par addition d'une solution 0,25 N de NaOH, en utilisant un appareil automatique de titration. Lorsque 12,5 ml de NaOH 0,25 N ont été consommés, on ajoute du bicarbonate de sodium jusqu'à pH = 8 et on extrait à l'éther éthylique puis on sépare les deux phases, on élimine la phase aqueuse contenant l'acide 6-méthoxy-1,2,3,4-tétrahydro-(2R)2-naphtoïque qui peut être récupéré, on sèche la phase organique sur du sulfate de sodium et on évapore le solvant sous pression réduite. On obtient 4,73 g de méthyl 6-méthoxy-1,2,3,4-tétrahydro-(2S)2-naphtoate (e.e. 92% - HPLC effectuée dans les conditions (a)). On traite l'isomère *(S)* de l'ester ainsi obtenu avec une solution de NaOH, on acidifie à l'aide d'acide chlorhydrique 1N et on obtient 3,2 g de l'acide indiqué en titre qu'on cristallise dans de l'acétate d'éthyle. P.f. 130-131 °C. Ce produit est plus pur que celui décrit dans EP-A-436 435, préparation (Q)(i).

## Exemple 3

### Acide 6-méthoxy-1,2,3,4-tétrahydro-(2S) 2-naphtoïque

On suit la méthode décrite dans l'Exemple 1, à partir de 4 g (0,018 mole) d'ester de départ au lieu de 3 g, en arrétant la réaction enzymatique lorsque 37 ml de NaOH 0,25 N ont été consommés, en additionnant du bicarbonate de sodium jusqu'à pH 8. Ensuite on extrait à l'éther éthylique, on sépare les deux phases, on sèche la phase organique sur du sulfate de sodium et on évapore le solvant sous pression réduite. On obtient 1,85 g de méthyl 6-méthoxy-1,2,3,4-tétrahydro-(2S)2-naphtoate (e.e. 92 % HPLC effectuée dans les conditions (a)). On traite l'isomère *(S)* de l'ester ainsi obtenu avec une solution de NaOH, on acidifie à l'aide d'acide chlorhydrique 1N et on obtient 1,6 g de l'acide indiqué en titre qu'on cristallise dans de l'acétate d'éthyle. P.f. 129-130 °C; $[\alpha]_{20}^{D}$ = - 57,3 ° (c. 1,4 % ,$CHCl_3$); e.e. 93,0 % (HPLC effectuée dans les conditions (a)).

## Exemple 4

### Acide 7-méthoxy-1,2,3,4-tétrahydro-(2R) 2-naphtoïque.

On dissout 5 g (0,022 mole) de méthyl 7-méthoxy-1,2,3,4-tétrahydro-2-naphtoate dans 175 ml de tert-butanol et on ajoute à la solution ainsi préparée 500 ml de tampon phosphate pH = 7. Le pH de la solution, qui augmente jusqu'à une valeur de 7,3 - 7,5, est ramené à 7,1 par addition de HCl 1N. On ajoute au mélange 3,5 g d'enzyme PPL Sigma (indiquée comme "porcine pancreatic lipase" type II, crude, Sigma L-3126 - 39 U/mg using triacetine). Au fur et à mesure que la réaction s'effectue, le pH tend à baisser mais on le maintient constant par addition d'une solution 0,25 N de NaOH. Après 6 heures, lorsque environ 31,75 ml de NaOH 0,25 N ont été consommés, on ajoute à la solution du bicarbonate de sodium jusqu'à obtenir pH ≈ 8, pour arrêter la réaction par inactivation de l'enzyme. On extrait dans de l'éther éthylique et on sépare les deux phases. La phase organique contient l'ester, principalement de configuration *(S)* qui n'a pas réagi et qui peut être récupéré comme indiqué dans l'Exemple 2. On acidifie la phase aqueuse à l'aide d'acide sulfurique concentré et on filtre le précipité formé. On obtient ainsi 1,25 g de l'acide indiqué en titre. e.e. 93,27 % (HPLC effectuée dans les conditions (a)). Ce produit est égal à celui décrit dans la Préparation (G)(i) de EP-A-436435, obtenu après 10 cristallisations. Deux autres préparations effectuées dans les mêmes conditions ont donné le même produit ayant un e.e. de 91,65% et 93,52% (HPLC effectuée dans les conditions (a)).

**Exemple 5**

**Acide 7-méthoxy-1,2,3,4-tétrahydro-(2S) 2-naphtoïque**

La phase organique résiduelle de l'Exemple 6 est séchée sur du sulfate de sodium et évaporée sous pression réduite. On obtient 3,39 g de l'ester contenant principalement l'isomère *(S)* (e.e. 37,6 % - HPLC effectuée dans les conditions (b)) qu'on distille (Eb = 130-135 ° C à 0,1 mbar). On dissout 3 g (0,014 mole) de l'ester ainsi récupéré dans 100 ml de *tert*-butanol; on ajoute à la solution ainsi obtenue 300 ml de tampon phosphate à pH7. On porte à pH 7,04 par addition d'acide sulfurique à 5% et on ajoute 2 g de PPL Sigma (indiquée comme "porcine pancreatic lipase" type II, crude, Sigma L-3126 - 39 U/mg using triacetine). Au fur et à mesure que la réaction s'effectue, le pH baisse; on le maintient constant par addition d'une solution 0,25 N de NaOH. Après 6 heures environ, lorsque 15ml de NaOH 0,25 N ont été consommés, on ajoute du bicarbonate de sodium jusqu'à pH = 8 et on extrait à l'éther éthylique puis on sépare les deux phases,on élimine la phase aqueuse contenant l'acide 7-méthoxy-1,2,3,4-tétrahydro-(2R)2-naphtoïque qui peut être récupéré, on sèche la phase organique sur du sulfate de sodium et on évapore le solvant sous pression réduite. On obtient 1,5 g de méthyl 7-méthoxy-1,2,3,4-tétrahydro-(2S)2-naphtoate (e.e. 92,3% - HPLC effectuée dans les conditions (b)). On traite l'isomère *(S)* de l'ester ainsi obtenu avec une solution de NaOH, on acidifie à l'aide d'acide chlorhydrique 1N et on obtient 1,1 g de l'acide indiqué en titre. e.e. 92,3% (c 1,4% $CHCl_3$). Ce produit est égal à celui décrit dans EP-A-436 435, obtenu après 10 cristallisations.

**Exemple 6**

**Chlorhydrate de 7-méthoxy-1,2,3,4-tétrahydro-(2R) 2-naphtaleneamine**

A une solution de 2,5 g (0,0120 mole) d'acide 7-méthoxy-1,2,3,4-tétrahydro-(2R)2-naphtoïque dans 30 ml d'acétone on ajoute lentement à la température de -10 ° C à -5 ° C, une solution de 2 ml (0,0140 mole) de triéthylamine dans 20 ml d'acétone et ensuite 1,6 ml (0,0161 mole) de chloroformiate d'éthyle dans 20 ml d'acétone. On agite à - 5 ° C pendant 2 heures puis on ajoute, goutte à goutte, une solution de 1,3 g (0,0193 mole) de $NaN_3$ dans 10 ml d'eau. Après 1 heure à - 5 ° C, on verse le mélange dans 200 ml d'eau et on extrait au toluène. On sépare les deux phases, on sèche la phase organique sur sulfate de sodium, on filtre et on chauffe la solution à 100 ° C pendant 1,5 heures. On évapore le solvant sous pression réduite, on reprend le résidu dans 22 ml d'eau et 26 ml d'acide chlorhydrique à 37 % et on chauffe au reflux pendant 3,5 heures. Après refioidissement, on porte la solution à pH basique par addition de NaOH 3,5 N, on extrait à l'éther éthylique, on sèche la phase organique sur sulfate de sodium et on évapore le solvant sous pression réduite. On prépare le chlorhydrate de la base ainsi obtenue à l'aide d'éther éthylique saturé d'acide chlorhydrique. On obtient 1,6 g du composé indiqué en titre qu'on cristallise dans de l'isopropanol. On obtient 1,2g de produit. P.f. 205 ° C-207 ° C; $[\alpha]_{20}^{D}$ = + 66,6 ° (c. 0,5% MeOH).

**Exemple 7**

**Chlorhydrate de 7-méthoxy-1,2,3,4-tétrahydro-(2S) 2-naphtaleneamine.**

On suit la méthode de l'Exemple 6 en utilisant 2,5 g d'acide 7-méthoxy-1,2,3,4-tétrahydro-(2S)2-naphtoïque en tant que produit de départ. On obtient 1 g du composé indiqué dans le titre P.f. 205 ° C - 207 ° C; $[\alpha]_{20}^{D}$ = - 66,4 ° (c. 0,4% MeOH).

**Exemple 8**

**(2R) 2-aminométhyl-6-méthoxy-1,2,3,4-tétrahydronaptalène**

(a) 6-méthoxy-1,2,3,4-tétrahydronaphtalene-(2R)2-carboxamide. A une solution de 7,01 g (0,034 mole) d'acide 6-méthoxy-1,2,3,4-tétrahydro-(2R)2-naphtoïque dans 150 ml de chlorure de méthylène, en conditions anhydres, on ajoute goutte à goutte 3,48 ml (0,038 mole) de chlorure d'oxalyle et quelques gouttes de diméthylformamide. On laisse agiter à la température ambiante pendant 5 heures puis on ajoute ce mélange, goutte à goutte, à une solution de 21,52 ml (0,102 mole) d'hexaméthyldisilazane dans 15 ml de chlorure de méthylène anhydre. On laisse agiter le mélange à la température ambiante pendant une nuit. On ajoute 25 ml de méthanol et on agite pendant 30 minutes. On verse dans 200 ml

d'acide sulfurique à 5% et on extrait au chlorure de méthylène. On sépare les deux phases, on lave la phase organique avec une solution saturée de chlorure d'ammonium, on sèche sur sulfate de sodium et on évapore le solvant sous pression réduite. On obtient 11,8 g de produit que l'on purifie par chromatographie flash en éluant avec un mélange chlorure de méthylène/méthanol = 9/1. On obtient 4,64 g de 6-méthoxy-1,2,3,4-tétrahydronaphtalene-(2R)2-carboxamide. e.e. 94,5% (HPLC effectuée dans les conditions (c)) $[\alpha]_{20}^{D}$ = +52,9° (c. 1,4% $CHCl_3$).
(b)on soumet le composé obtenu dans l'étape (a) ci-dessus à la réaction décrite dans EP-A-436435, Préparation (O) (iii). On obtient le (2R)2-aminométhyl-6-méthoxy-1,2,3,4-tétrahydronaphtalène.

## Revendications

**1.** Procédé pour la préparation des composés de formule (I) sous forme optiquement active

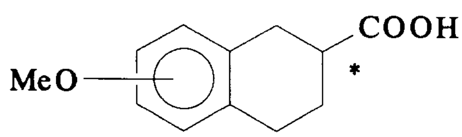
(I)

caractérisé en ce que :
(a) on soumet un ester racémique de formule

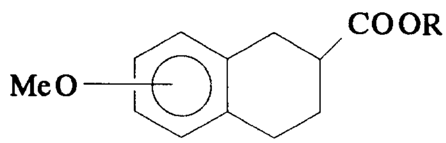
(II)

dans laquelle R est un alkyle en $C_1$-$C_3$, à une hydrolyse par une lipase; puis
(b) lorsque environ 50 % de l'ester est hydrolysé en acide, on interrompt l'hydrolyse par inactivation de l'enzyme et on récupère l'ester de configuration *(S)* de formule

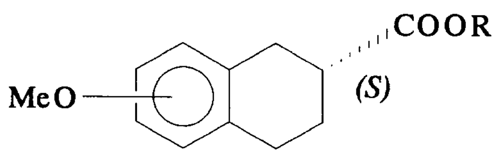
(II')

qui n'a pas réagi, enfin
(c) soit on hydrolyse ledit ester de formule (II') et on isole l'acide de configuration *(S)* de formule

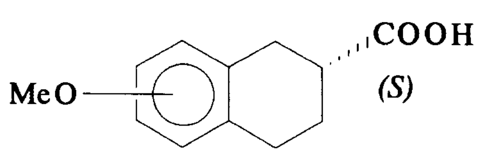
(I')

soit on isole l'acide de configuration *(R)* de formule

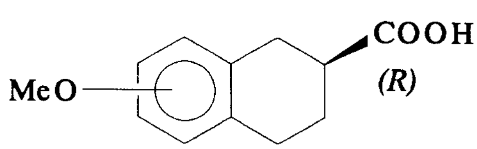
(I")

tel qu'obtenu de la réaction d'hydrolyse lipasique après interruption de l'hydrolyse à l'étape (b).

**2.** Procédé selon la revendication 1, caractérisé en ce que dans l'étape (b) on interrompt l'hydrolyse enzymatique juste avant que 50% du composé (II) soit hydrolysé, par addition d'hydroxyde de sodium jusqu'à pH basique, on extrait le composé (II') qui n'a pas réagi dans un solvant organique immiscible à l'eau puis on élimine la phase organique et, dans l'étape (c) on traite la phase aqueuse avec un acide minéral ou organique et on sépare l'acide de configuration *(R)* de formule (I'') ainsi précipité.

**3.** Procédé selon la revendication 1, caractérisé en ce que dans l'étape (b) on interrompt l'hydrolyse enzymatique juste après que 50% du composé (II) soit hydrolysé, par addition d'hydroxyde de sodium jusqu'à pH basique, on extrait le composé (II') qui n'a pas réagi dans un solvant organique immiscible à l'eau puis on élimine la phase aqueuse, on isole l'ester (II') et, dans l'étape (c) on hydrolyse ledit ester de formule (II') et on isole l'acide de configuration *(S)* de formule (I').

**4.** Procédé selon l'une quelconque des revendications 1 à 3, caractérisé en ce que l'étape (a) est effectuée en milieu aqueux.

**5.** Procédé selon l'une quelconque des revendications 1 à 4, caractérisé en ce que la lipase utilisée est la lipase de pancréas de porc.

**6.** Procédé selon l'une quelconque des revendications 1 à 5, caractérisé en ce que la réaction est effectuée en milieu aqueux en présence d'un tampon à pH 7.

**7.** Procédé selon l'une quelconque des revendications 1 à 6, caractérisé en ce que la température de réaction de l'étape (a) est comprise entre 0 °C et +60 °C.

**8.** Procédé selon l'une quelconque des revendications 1 à 7, caractérisé en ce que comme produit de départ, on utilise un composé de formule (II) dans laquelle R est méthyle.

**9.** Composé de formule

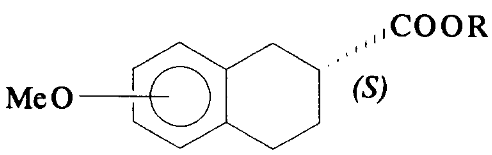

(II')

dans laquelle R est un alkyle en $C_1$-$C_3$.

**10.** 6-méthoxy-1,2,3,4-tétrahydro-(2S)2-naphtoate de méthyle

**11.** 7-méthoxy-1,2,3,4-tétrahydro-(2S)2-naphtoate de méthyle.

Office européen des brevets

# RAPPORT DE RECHERCHE EUROPEENNE

Numero de la demande
EP 94 40 0863

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | DE-A-33 45 660 (BOEHRINGER) 27 Juin 1985<br>* revendications *<br>--- | 1 | C12P41/00<br>C07C62/14<br>C12P7/40 |
| D,A | EP-A-0 300 404 (SEARLE) 25 Janvier 1989<br>* revendications; exemple 2 *<br>--- | 1 | |
| D,A | EP-A-0 436 435 (SANOFI) 10 Juillet 1991<br>* page 16 - page 23; revendications *<br>----- | 1,9-11 | |
| | | | **DOMAINES TECHNIQUES RECHERCHES (Int.Cl.6)** |
| | | | C12P<br>C07C |

Le présent rapport a été établi pour toutes les revendications

| Lieu de la recherche | Date d'achèvement de la recherche | Examinateur |
|---|---|---|
| LA HAYE | 26 Septembre 1994 | Delanghe, L |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..............................
& : membre de la même famille, document correspondant